# EUROPÄISCHE PATENTSCHRIFT

(11) **EP 0 513 054 B1**
(45) Veröffentlichungstag und Bekanntmachung des Hinweises auf die Patenterteilung: **21.04.1999**
(21) Anmeldenummer: 91902693.0
(22) Anmeldetag: 24.01.1991
(51) Int. Cl.: C12N 15/40, C12N 15/54, C12N 5/10, A01H 5/00

(54) **VIRUS/HERBIZIDRESISTENZ-GENE, VERFAHREN ZU IHRER HERSTELLUNG UND IHRE VERWENDUNG**
VIRUS/HERBICIDE RESISTANCE GENES PROCESS FOR PRODUCING SAME AND THEIR USE
GENES DE RESISTANCE AUX VIRUS/HERBICIDES, LEUR PROCEDE DE PRODUCTION ET LEUR UTILISATION

(30) Priorität: 02.02.1990 DE 4003045
(43) Veröffentlichungstag der Anmeldung: 19.11.1992
(60) Teilanmeldung: 98108424.7
(73) Patentinhaber: Hoechst Schering AgrEvo GmbH, 13342 Berlin (DE)
(72) Erfinder: SCHNEIDER, Rudolf, D-6233 Kelkheim (DE); DONN, Günter, D-6238 Hofheim am Taunus (DE); MÜLLNER, Hubert, D-6233 Kelkheim (DE)
(86) Internationale Anmeldenummer: EP9100130
(87) Internationale Veröffentlichungsnummer: WO9111517

(56) Entgegenhaltungen:
- EP-A- 240 331
- EP-A- 275 957
- EP-A- 298 918
- The EMBO Journal, Band 6, Nr. 7, 1987, IRL Press Ltd (Oxford, GB) L.S. Loesch-Fries et al.: "Expression of alfalfa mosaic virus RNA 4 in transgenic plants confers virus resistance", Seiten 1845 - 1851
- Chemical Abstracts, Band 112, 1990 (Columbus, Ohio, US) J. Botterman et al.: "Discovery, transfer to crops, expression and biological significance of a bialaphos resistance gene", siehe Seite 116

## Beschreibung

Die Synthese von Virus "coat" Protein in Pflanzen führt zu einer verstärkten Resistenz der Pflanze gegenüber dem entsprechenden Virus. Die Europäische Patentanmeldung 0 240 331, beispielsweise, beschreibt die Herstellung von Pflanzen-Zellen, die ein solches "coat" Protein enthalten.

Tumer et al. [EMBO J. 6, 1181 (1987)] haben die Transformation von Tabak- und Tomaten-Pflanzen mit einem chimären Gen, das für das "coat" Protein des Alfalfa Mosaic Virus kodiert, durchgeführt. Die Nachkommen dieser transformierten Pflanzen zeigten eine signifikante Reduzierung der entsprechenden Virus-Infektionssymptome, in einigen Fällen sogar Virus-Resistenz.

Es wurde nun gefunden, daß solche Virusgene mit einem Herbizidresistenzgen kombiniert werden können, was die Selektion der transgenen Pflanzen erleichtert. Gleichzeitig wird im praktischen Feldanbau die Vitalität der Pflanzen durch die Virustoleranz gesteigert und durch das Herbizidresistenzgen ein verbesserter Pflanzenschutz möglich. Es wurde allgemein beobachtet, daß die Herbizidapplikation einen stimulierenden Einfluß auf das Wachstum ausübt. Die erfindungsgemäß transformierte Pflanze zeigt diesen Effekt verstärkt, wodurch ein verbesserter Pflanzenertrag erreicht werden kann.

Herbizidresistenzgene sind bereits bekannt. In der Offenlegungschrift DE 37 16 309 wird die Selektion nicht-pilzähnlicher Bakterien, die gegen Phosphinothricin resistent sind, beschrieben. Aus der DNA dieser Selektanten kann das Phosphinothricin-Resistenzgen auf ein 2 kb großes Fragment eingegrenzt werden.

In der Deutschen Offenlegungsschrift 37 37 918 ist ein Weg zur Synthese des Phosphinothricin-Resistenzgens aus dem Genom von Streptomyces viridochromogenes aufgezeigt. Ein Einbau in Genstrukturen, mit Hilfe derer transformierte Pflanzen gegen das Herbizid resistent werden, wird dort ebenfalls beschrieben.

Die Erfindung betrifft somit Pflanzenzellen, enthaltend ein Gen kodierend für eine Phosphinothricinresistenz und ein Gen kodierend für eine Virusresistenz. Im folgenden wird die Erfindung detailliert beschrieben, insbesondere in ihren bevorzugten Ausführungsformen. Ferner wird die Erfindung durch den Inhalt der Ansprüche bestimmt.

Die Gene für die Virusresistenz, insbesondere die Virus "coat" Proteine, kann man ausgehend von isolierter Virus RNA durch cDNA-Klonierung in Wirtsorganismen erhalten. Bevorzugt wird dabei von der RNA des Cucumber Mosaic Virus, des Alfalfa Mosaic Virus oder des Brom Mosaic Virus ausgegangen.

Herbizidresistenzgene können aus Bakterien, z.B. der Gattungen Streptomyces oder Alcaligenes, isoliert werden. Bevorzugt wird mit dem Phosphinothricinresistenzgen aus Streptomyces viridochromogenes (Wohlleben, W. et al., Gene 80, 25-57 (1988)) gearbeitet, das für eine Expression in Pflanzen entsprechend modifiziert werden kann.

Die Gene werden jeweils mit Hilfe der Vektoren pUC19, pUC18 oder pBluescript (Stratagene, Heidelberg, Product Information) kloniert und sequenziert.

Das Gen wird in einen intermediären Vektor mit Pflanzenpromotor kloniert. Derartige Vektoren sind beispielsweise die Plasmide pPCV701 (Velten J. et al. EMBO J. 3, 2723-2730 (1984)), pNCN (Fromm H. et al. PNAS 82, 5824-5826 (1985)), oder pNOS (an, G. et al., EMBO J. 4, 277-276 (1985)). Bevorzugt wird der Vektor pDH51 (Pietrzak, M. et al., NAR 14, 5857, (1986)) mit einem 35S-Promotor, bzw. der Vektor pNCN mit einem Nos-Promotor verwendet.

Nach anschließender Transformation von E. coli, wie z.B E. coli MC 1061, DH1, DK1, GM48 oder XL-1, werden positive Klone nach an sich bekannten Methoden (Maniatis et al., Lab. Manual), wie Plasmidminipräparation und Spaltung mit einem entsprechenden Restriktionsenzym, identifiziert.

Diese positiven Klone werden dann zusammen in einen binären Pflanzenvektor subkloniert. Als Pflanzenvektor können pGV3850 (Zambrysk, P. et al., EMBO J. 2, 2143-2150 (1983)) oder pOCA18 (Olszewski, N., NAR 16, 10765-10782, (1988)) eingesetzt werden. Vorteilhaft wird mit pOCA18 gearbeitet.

Die erhaltenen binären Pflanzenvektoren, die Pflanzenpromotoren mit dem angehängten DNA-Fragment für die Expression von Virus Coat Protein und Phosphinthricin Resistenz in der T-DNA enthalten, werden verwendet, um Pflanzen zu transformieren. Dies kann durch Techniken wie Elektroporation oder Mikroinjektion erfolgen. Bevorzugt wird die Kokultivierung von Protoplasten oder die Transformation von Blattstückchen mit Agrobakterien angewandt. Dazu wird das Pflanzenvektorkonstrukt durch Transformation mit gereinigter DNA oder, vermittelt über einen Helferstamm wie E. coli SM10 (Simon R. et al., Biotechnology 1, 784-791 (1983)) in Agrobakterium tumefaciens wie A282 mit einem Ti Plasmid über ein "triparental mating" transferiert. Direkte Transformation und triparental mating wurden, wie in "Plant Molecular Biology Manual" (Kluwer Academic Publisher, Dardrech (1988)) beschrieben, durchgeführt.

Es können grundsätzlich alle Pflanzen mit den die erfindungsgemäß konstruierte DNA tragenden binären Pflanzenvektoren transformiert werden. Bevorzugt sind dikotyledone Pflanzen, insbesondere Nutzpflanzen, die Stärke, Kohlenhydrate, Eiweiße oder Fette in nutzbaren Mengen in ihren Organen produzieren oder speichern oder die Obst und Gemüse produzieren oder die Gewürze, Fasern und technisch verwertbare Produkte oder Arzneimittel, Farbstoffe oder Wachse liefern und ebenfalls Futterpflanzen. Als Beispiel sollen Tomate, Erdbeere, Avocado sowie Plfanzen, die tropische Früchte tragen, z.B. Papaya, Mango, aber auch Birne, Apfel, Nektarine, Aprikose oder Pfirsich genannt werden. Ferner sollen als zu transformierende Pflanzen beispielhaft alle Getreidearten, Raps, Rüpsen... angeführt werden. Die transformierten Zellen werden mit Hilfe eines Selektionsmediums selektiert, zu einem Kallus herangezüchtet und auf einem entsprechenden Medium zur Pflanze regeneriert (Shain M. et al., Theor. appl. Genet. 72, 770-770 (1986); Masson, J. et al., Plant Science 53, 167-176 (1987), Zhan X. et al., Plant Mol. Biol. 11, 551-559 (1988); McGranaham G. et al., Bio/Technology 6, 800-804 (1988); Novak F. J. et al., Bio/Technology 7, 154-159 (1989).

Die folgenden Beispiele dienen der weiteren Erläuterung der Erfindung.

### Beispiele

### 1. Isolierung des Virus coat Protein Gens

Die Reinigung des Virus erfolgte nach einer modifizierten Methode von Lot, M. et al. Anual Phytopath. 4, 25-32 (1972). Luzerne wurden mit Alfalfa Mosaic Virus infiziert und nach 14 Tagen im gleichen Volumen 0,5 M Na-Citrat (pH 6,5)/ 5 mM EDTA/0,5 % Thioglykolsäure aufgeschloßen. Dann gab man 1 Volumen Chloroform hinzu und zentrifugierte 10 Min mit 12000 x g. Der Überstand wurde mit 10 % PEG 6000 (w/w) versetzt und über Nacht vorsichtig gerührt. Danach wurde 10 Min mit 12000 x g abzentrifugiert und in 50 ml 5 mM Na-Borat, 0,5 mM EDTA (pH 9) resuspendiert. Nach Zugabe von Triton-X-100 (2 % Endkonzentrationen) wurde 30 Min gerührt und mit 19000 x g 15 Min abzentrifugiert. Das Virussediment wurde nach Zentrifugation mit 105000 x g 2 Std. in 5 mM Borat Puffer/0,5 mM EDTA (pH 9,0) aufgenommen und einer Saccharosezentrifugation (5-25 %) unterworfen.

Einzelne Fraktionen des Gradienten wurden auf einem Agarosegel analysiert, um den virushaltigen Bereich zu ermitteln. Die Virus RNA wurde durch Phenol/SDS Extraktion (Peden, K.W. et al., Virology 53, 487-492 (1973) von coat" Protein gereinigt. Die Auftrennung der RNA-Komponenten erfolgte mit Hilfe von 2,8 % Polyacrylamid mit 40 mM Trisacetatpuffer (pH 7,5) wie in Synous, R.H., Aust. J. Biol. Sci 31, 25-37 (1978) beschrieben. Die RNA wurde elektrophoretisch in Dialyseschläuchen aus dem Gel entfernt und gefällt.

cDNA Transkripte von RNA3 bzw. RNA4 wurden, wie in Langenreis, K. et al. Plant Mol. Biol. 6, 281-288 (1986) beschrieben, mit Hilfe von synthetischen Oligonukleotidprimern mit 3'-komplementären Nukleotiden zum Template, die am 5'-Ende jeweils eine SmaI bzw. PstI Schnittstelle besaßen, hergestellt.

Die Reaktionen zur cDNA Synthese erfolgten nach den "Current Protocols in Mol. Biol." ed. Ausubel, F. et al., John Wiley and Sons.

Die cDNA wurde in den SmaI/PstI geschnittenen pUC 19 Vektor kloniert. Die Insertion konnte mit SmaI/HindIII wieder entfernt werden.

Die vorgehend beschriebene Methode kann ebenso für die Isolierung des CMV "coat" protein Gens angewendet werden.

### 2. Isolierung des Herbizidresistenz Gens

Es wurde ein Phosphinothricinresistenzgen mit folgender Sequenz nach der Phosphoamidit-Methode in einem Synthesizer synthetisiert.

9 18 27 36 45
5' GTC GAC ATG TCT CCG GAG AGG AGA CCA GTT GAG ATT AGG CCA GCT
3' G TAC AGA GGC CTC TCC TCT GGT CAA CTC TAA TCC GGT CGA
54 63 72 81 90
ACA GCA GCT GAT ATG GCC GCG GTT TGT GAT ATC GTT AAC CAT TAC
TGT CGT CGA CTA TAC CGG CGC CAA ACA CTA TAG CAA TTG GTA ATG
99 108 117 126 135
ATT GAG ACG TCT ACA GTG AAC TTT AGG ACA GAG CCA CAA ACA CCA
TAA CTC TGC AGA TGT CAC TTG AAA TCC TGT CTC GGT GTT TGT GGT
144 153 162 171 180
CAA GAG TGG ATT GAT GAT CTA GAG AGG TTG CAA GAT AGA TAC CCT
GTT CTC ACC TAA CTA CTA GAT CTC TCC AAC GTT CTA TCT ATG GGA
189 198 207 216 225
TGG TTG GTT GCT GAG GTT GAG GGT GTT GTG GCT GGT ATT GCT TAC
ACC AAC CAA CGA CTC CAA CTC CCA CAA CAC CGA CCA TAA CGA ATG
234 243 252 261 270
GCT GGG CCC TGG AAG GCT AGG AAC GCT TAC GAT TGG ACA GTT GAG
CGA CCC GGG ACC TTC CGA TCC TTG CGA ATG CTA ACC TGT CAA CTC
279 288 297 306 315
AGT ACT GTT TAC GTG TCA CAT AGG CAT CAA AGG TTG GGC CTA GGA
TCA TGA CAA ATG CAC AGT GTA TCC GTA GTT TCC AAC CCG GAT CCT
324 333 342 351 360
TCC ACA TTG TAC ACA CAT TTG CTT AAG TCT ATG GAG GCG CAA GGT
AGG TGT AAC ATG TGT GTA AAC GAA TTC AGA TAC CTC CGC GTT CCA
369 378 387 396 405
TTT AAG TCT GTG GTT GCT GTT ATA GGC CTT CCA AAC GAT CCA TCT
AAA TTC AGA CAC CAA CGA CAA TAT CCG GAA GGT TTG CTA GGT AGA
414 423 432 441 450
GTT AGG TTG CAT GAG GCT TTG GGA TAC ACA GCC CGG GGT ACA TTG
CAA TCC AAC GTA CTC CGA AAC CCT ATG TGT CGG GCC CCA TGT AAC
459 468 477 486 495
CGC GCA GCT GGA TAC AAG CAT GGT GGA TGG CAT GAT GTT GGT TTT
GCG CGT CGA CCT ATG TTC GTA CCA CCT ACC GTA CTA CAA CCA AAA
504 513 522 531 540
TGG CAA AGG GAT TTT GAG TTG CCA GCT CCT CCA AGG CCA GTT AGG
ACC GTT TCC CTA AAA CTC AAC GGT CGA GGA GGT TCC GGT CAA TCC
549 558
CCA GTT ACC CAG ATC TGA G 3'
GGT CAA TGG GTC TAG ACT CAG CTG 5'

Es handelt sich hierbei um eine Modifikation der von Wohlleben in Gene 70, 25-37 (1988) veröffentlichten Sequenz für das Acetyltransferasegen.

Es ist ebenfalls möglich, eine genomische DNA-Bank aus dem von Wohlleben benutzten Streptomyces viridochromogenes in EMBL3 in E. coli auf Acetylierung von Phosphinothricin zu untersuchen. Das acetylierte Produkt kann sehr leicht dünnschichtchromatographisch aufgetrennt werden.

Das Gen wurde in pUC19 kloniert und sequenziert. Die Expression in Pflanzen erfolgte als SalI-Fragment.

### 3. Fusion Herbizidresistenzgen mit Nos Promotor

Der Vektor pNCN wurde Bam/SalI verdaut und das entstehende 2,5 bp Stück isoliert. Die überstehenden Enden wurden mit Mung bean Nuclease abverdaut. Das Acetyltransferase Gen wurde als 0,5 bp Stück nach SalI Verdau isoliert und mit Klenow aufgefüllt. Nach Ligase konnten positive Klone durch Plasmidminipräparationen isoliert werden. Die Orientierung ergab sich aus einem SalI/Bam Verdau.

### 4. Fusion "coat" Protein Gen mit 35S Promotor

Ein 0,5 Basenpaare langes Fragment aus pAI RNA3 (dem pUC19 Vektor mit "coat" Protein Gen Insert) wurde nach SmaI/Hind III Verdau isoliert. Die überstehenden Enden wurden durch Mung bean Nuclease abverdaut. Der Vektor pDH 51 wurde mit XbaI geschnitten und die Enden mit Klenow-Polymerase aufgefüllt. Fragment und Vektor wurden ligiert und in MC 1061 transformiert (p35/AI). Dieselbe Konstruktion wurde mit pCM RNA3 für das "coat". Protein von CMV geschaffen (p35/CM).

### 5. Fusion von 35S/"coat" Protein Gen und nos/Acetyltransferase Gen

Ein 1,3 kb Stück aus dem 35S/"coat" Protein Konstrukt (p35/AI, p35/CM) wurde nach EcoRI Verdau aus einem "low melt" Agarose Gel isoliert. Der Pflanzenvektor pOCA 18 wurde EcoRI verdaut und mit dem 1,3 kbp DNA Stück ligiert. Dieser pOCA/35 RNA3 Vektor wurde mit Klenow aufgefüllt. Ein 2,5 kbp Hind III Stück aus nos/AC wurde nach Klenow Behandlung der Enden in die aufgefüllte ClaI Stelle eingesetzt.

Konstruktionen: pOCA/AcAI3
pOCA/AcCM3

### 6. Transformation von Agrobakterien

Der Agrobakterienstamm pMP90RK wurde mit pOCA/AcAI3 bzw. pOCA/AcCM3 im triparental mating mit SM10 transformiert. Je 100 µl Bakterien aus Übernachtkulturen von SM10, die die Konstruktion tragenden MC 1061 und der Agrobakterien wurden abzentrifugiert und zusammen in 30 µl LB Medium suspendiert. Diese Zellen gab man auf ein kleines Rundfilter auf einer LB-Platte ohne Antibiotikum. Nach 12 Std. Inkubation bei 37°C wurde der Filter in 2,5 ml 10 mM MgCl₂ gewaschen und Aliquots davon auf LB-Platten mit Rifampicin, Tetrazyklin und Kanamycin selektiert. Positive Kolonien wurden durch Hybridisierung mit ³²P markierter DNA der Gene identifiziert.

### 7. Transformation von Luzerne

Zur Transformation der Luzerne wurde eine modifizierte Version der Kokultivierungsmethode nach Marton S. et al., Nature 277, 129-130 (1979) eingesetzt. Etwa 1 cm lange Stengelabschnitte von sterilen Pflanzen wurden in Erlenmeyerkolben in 40 ml steriles MS Medium gegeben und 11 ml einer verdünnten Übernachtkultur der Agrobakterien (5 x 10⁷ Zellen/ml) zugegeben. Die Inkubation wurde 3 Tage bei 25°C fortgesetzt. Die Stengelsegmente wusch man anschließend dreimal mit sterilem Wasser und brachte sie auf MS-Medium mit 300 mg/l Carbamicillin und 100 mg/l Kanamycin. Nach 3 Wochen bildete sich ein Kallus, aus dem ganze Pflanzen regeneriert werden konnten.

### 8. Test der Pflanzen

Die Pflanzen zeigten nach Aufarbeitung von RNA und Hybridisierung mit radioaktiv markierter DNA der Gene Expression von AC-Gen mit Alfalfa Mosaic Virus "coat" Protein Gen.

Die Pflanzen wuchsen auf phosphinothricinhaltigem Medium und zeigten deutliche Toleranz nach Infektion mit Alfalfa Mosaic Virus.

## Patentansprüche

1. Pflanzenzellen, enthaltend ein Gen kodierend für eine Phosphinothricinresistenz und ein Gen kodierend für eine Virusresistenz.

2. Pflanzenzellen gemäß Anspruch 1, dadurch gekennzeichnet, daß das Virusresistenzgen für ein Virus-Hüllprotein kodiert.

3. Pflanzenzellen gemäß Anspruch 1 oder 2, dadurch gekennzeichnet, daß der für die Virusresistenz verantwortliche Teil des Gens durch cDNA-Klonierung ausgehend von der RNA der Cucumber-Mosaik-Virus, Alfalfa-Mosaik-Virus oder des Brom-Mosaik-Virus erhältlich ist.

4. Pflanzenzellen gemäß Anspruch 1 oder 2, dadurch gekennzeichnet, daß die Phosphinothricinresistenz aus Streptomyceten verwendet wird.

5. Verfahren zur Herstellung von Pflanzen mit verbesserten Eigenschaften, dadurch gekennzeichnet, daß aus Pflanzenzellen gemäß Anspruch 1 Pflanzen regeneriert werden und diese Pflanzen mit Phosphinothricin behandelt werden.

6. Verwendung einer Pflanzenzelle gemäß Anspruch 1 zur Regeneration von Pflanzen.

7. Verwendung einer Pflanzenzelle gemäß Anspruch 1 zur Regeneration von Pflanzen mit verbesserten Eigenschaften.

## Claims

1. Plant cells comprising a gene coding for a phosphinothricin resistance and a gene coding for a virus resistance.

2. Plant cells as claimed in claim 1, wherein the virus resistance gene codes for a virus coat protein.

3. Plant cells as claimed in claim 1 or 2, wherein the part of the gene responsible for the virus resistance can be obtained by cDNA cloning starting from the RNA of cucumber mosaic virus, alfalfa mosaic virus or of brom mosaic virus.

4. Plant cells as claimed in claim 1 or 2, wherein the phosphinothricin resistance from Streptomyces is used.

5. A process for producing plants with improved properties, which comprises plants being regenerated from plant cells as claimed in claim 1, and these plants being treated with phosphinothricin.

6. The use of a plant cell as claimed in claim 1 for the regeneration of plants.

7. The use of a plant cell as claimed in claim 1 for the regeneration of plants with improved properties.

## Revendications

1. Cellules végétales, contenant un gène codant pour une résistance à la phosphinothricine et un gène codant pour une résistance aux virus.

2. Cellules végétales selon la revendication 1, caractérisées en ce que le gène de résistance aux virus code pour une protéine de l'enveloppe du virus.

3. Cellules végétales selon la revendication 1 ou 2, caractérisées en ce que la partie du gène responsable de la résistance aux virus peut être obtenue par clonage d'ADNc en partant de l'ARN du virus de la mosaïque du concombre, du virus de la mosaïque de l'alfalfa ou du virus de la mosaïque du brome.

4. Cellules végétales selon la revendication 1 ou 2, caractérisées en ce qu'on utilise la résistance à la phosphinothricine issue de Streptomycètes.

5. Procédé de préparation de plantes ayant des propriétés améliorées, caractérisé en ce que, l'on régénère des plantes à partir des cellules végétales selon la revendication 1 et que ces plantes sont traitées avec de la phosphinothricine.

6. Utilisation d'une cellule végétale selon la revendication 1 pour la régénération de plantes.

7. Utilisation d'une cellule végétale selon la revendication 1 pour la régénération de plantes ayant des propriétés améliorées.
